Europäisches Patentamt

European Patent Office

Office européen des brevets

(11) Publication number: 0 374 817 A2

# EUROPEAN PATENT APPLICATION

(21) Application number: 89123429.6

(51) Int. Cl.⁵: C12N 7/00, //A61K39/12

(22) Date of filing: 19.12.89

(30) Priority: 21.12.88 JP 323951/88

(43) Date of publication of application:
27.06.90 Bulletin 90/26

(84) Designated Contracting States:
AT BE CH DE ES FR GB GR IT LI LU NL SE

(71) Applicant: TAKEDA CHEMICAL INDUSTRIES, LTD.
3-6, Doshomachi 2-chome Chuo-ku
Osaka 541(JP)

(72) Inventor: Koseki, Isao
259-3, Oaza-ohano Tabuse-cho
Kumage-gun Yamaguchi 742-15(JP)
Inventor: Kanno, Masayoshi
10-27, Mitsui 8-chome
Hikari Yamaguchi 743(JP)

(74) Representative: von Kreisler, Alek,
Dipl.-Chem. et al
Patentanwälte Von Kreisler-Selting-Werner
Deichmannhaus am Hauptbahnhof
D-5000 Köln 1(DE)

(54) Method for disrupting virus particles.

(57) Disclosed is a method for disrupting virus particles comprising subjecting the virus particles to high-seed collision treatment, preferably using a Mircofluidizer (registered trade mark), thereby being capable of disrupting the virus particles without damaging the partial structure of viral envelopes thereof, which results in the preparation of an influenza vaccine high in immunogenicity (potency) and having no pyrogenicity.

EP 0 374 817 A2

## METHOD FOR DISRUPTING VIRUS PARTICLES

BACKGROUND OF THE INVENTION

The present invention relates to a method for disrupting virus particles.

Disrupting methods of virus particles previously used include chemical disrupting methods such as ether treatment and physical disrupting methods such as ultrasonic treatment. By these methods, however, it was impossible to disrupt the virus particles leaving the partial structure of viral envelopes unruptured.

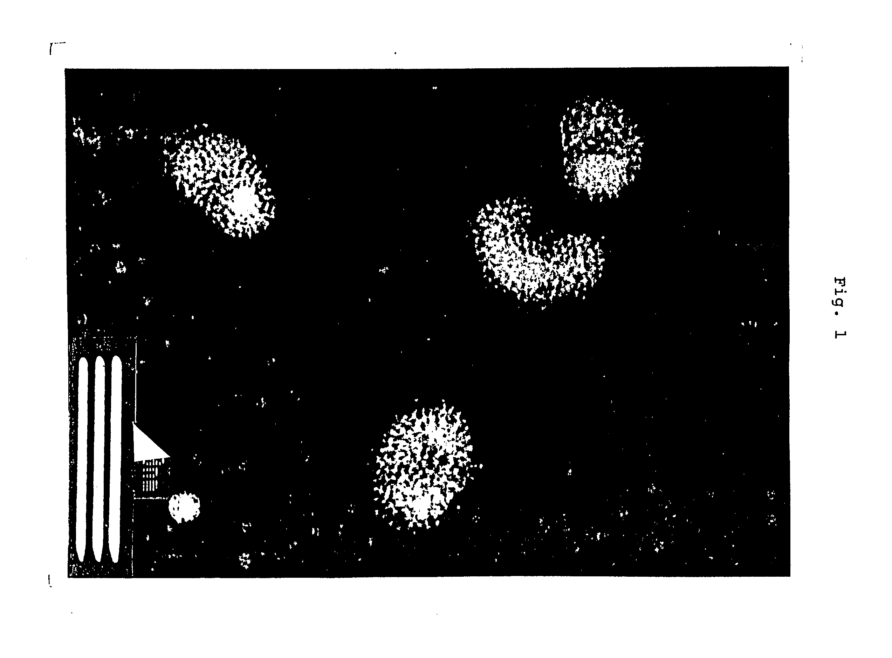

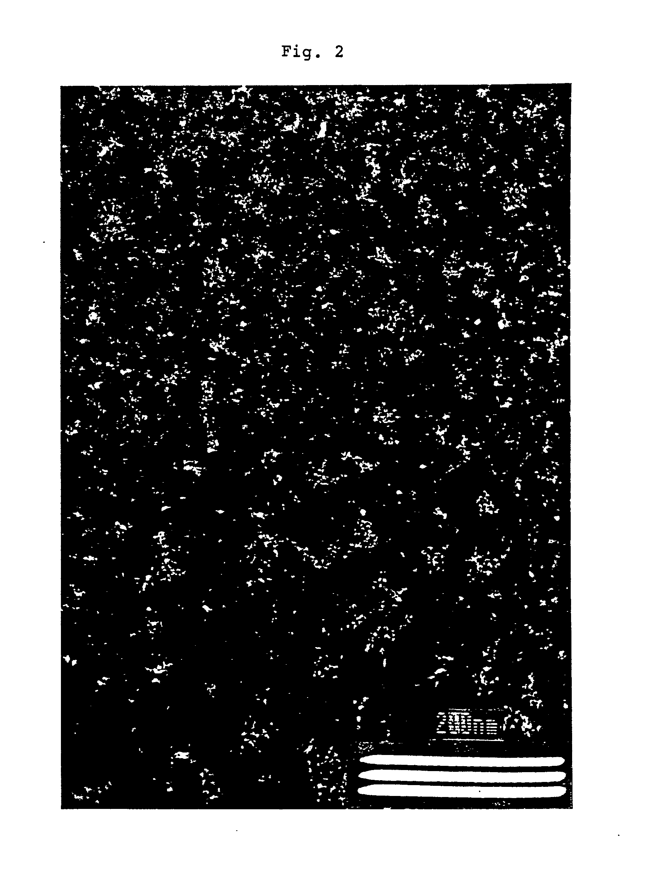

This will hereinafter be described, giving an example of virus disrupting methods used in the manufacturing processes of influenza vaccines. In the manufacture of the existing influenza HA vaccine (see Fig. 2) developed in the place of whole particle vaccines (see Fig. 1) in which a problem was encountered in side effects such as pyrogenicity, there is used the method of treating influenza virus particles with ether to disrupt the virus particles, thereby allowing hemoagglutinin (hereinafter referred to as HA) and neuraminidase (hereinafter referred to as NA) which are protective antigens of the influenza virus to act as immunogens. According to this method, the structure of a viral envelope which is formed by HA and NA externally bonded to a surface layer of the influenza virus particle, namely to an outer layer of a lipid bilayer and an inner layer of a matrix protein adjacent to it is broken. In the existing influenza HA vaccine manufactured by using this method, it is known that the pyrogenicity disappears, but that the immunogenicity (potency) of HA and NA is reduced due to the breakage of their bonding to the lipid bilayer and the matrix protein. It is therefore desired to enhance the immunogenicity (potency) under the present conditions. Methods for physically disrupting the influenza virus particles by ultrasonic treatment have also been attempted. It is however impossible to disrupt the influenza virus particles without damaging the partial structure of the viral envelopes thereof, as with the above ether treatment, though these methods are suitable for collection of HA and NA.

To enhance the immunogenicity (potency) of the existing influenza HA vaccine, various attempts have been made such as adding adjuvants (immunological activating agents) to HA and NA decomposed by the ether treatment and bonding HA and NA decomposed by the ether treatment to an artificial envelope formed of a lipid component to reconstruct the whole particle type of vaccine. However, these attempts are still outside the realm of practical satisfaction.

On the other hand, the principle of high-speed collision has previously been applied to the disruption of cells having a diameter of about 10 to 30 $\mu$m. However, it has not been considered at all to apply this principle to the disruption of virus particles having a diameter of about 18 to 600 nm, because the apparatus has limitations in pressure, flow rate and velocity during the treating process.

SUMMARY OF THE INVENTION

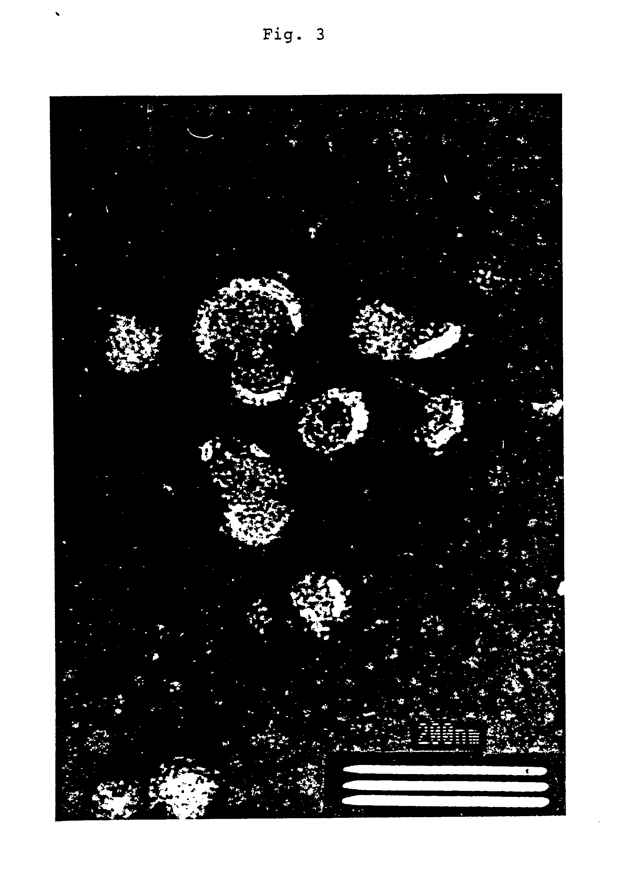

The present inventors have unexpectedly discovered that the influenza virus particles are disrupted without damaging the partial structure of the viral envelopes, when the virus particles are allowed to collide with one another at high speed by the application of high pressure (see Fig. 3). Further, the present inventors have discovered that an influenza vaccine which has the immunogenicity (potency) equivalent to that of the whole particle vaccine and whose pyrogenicity disappears can be produced by using the disrupted virus particles thus obtained.

In accordance with the present invention, there is provided a method for disrupting virus particles which comprises subjecting the virus particles to high-speed collision treatment.

BRIEF DESCRIPTION OF THE DRAWINGS

Fig. 1 is an electron microphotograph showing the configuration of virus (organism) particles in an original fluid of inactivated intact vaccine (whole particle vaccine) prepared in Example 4;

Fig. 2 is an electron microphotograph showing the configuration of disrupted virus (organism) particles in original fluid of an ether-treated vaccine (HA vaccine) prepared in Example 4; and

Fig. 3 is an electron microphotograph showing the configuration of disrupted virus (organism) particles in original fluid of a Microfluidizer-treated vaccine prepared in Example 4.

The white lines shown at the lower right of Figs. 1 to 3 indicate a length of 200 nm.

2

## DESCRIPTION OF THE PREFERRED EMBODIMENTS

Examples of the virus particles used in the present invention include influenza virus particles having a diameter of about 20 to 300 nm, influenza virus particles rubella virus particles, Japanese encephalitis virus particles, mumps virus particles, measles virus particles, adenovirus particles, herpes simplex virus particles, rabies virus particles, vaccinia virus particles, hepatitis B virus particles, hepatitis A virus particles, adult T cell leukemia virus particles and human immunodeficiency virus particles.

A material to be subjected to the high-speed collision treatment is prepared from the virus particles described above and a suitable medium, desirably a liquid. Methods for preparing the material to be subjected to the high-speed collision treatment will hereinafter be described by means of examples in which the influenza virus particles are used as the virus particles.

An influenza virus strain for manufacturing the influenza vaccine is inoculated, for example, in a chorioallantoic cavity of a grown egg and the virus is cultivated to obtain virus suspension. Examples of such influenza virus strains include A/Yamagata/120/86(H1N1) strain, A/Yamanashi/2/77(H3N2) strain, A/Fukuoka/C29/85/(H3N2) strain, A/Shisen/2/87(H3N2) strain, B/Gifu/2/73 strain, B/Kanagawa/3/76 strain and B/Nagasaki/1/87 strain. The virus suspension thus obtained may be used as the material for the high-speed collision treatment as it is. It is however desirable to use a fractionated virus fluid collected by further purifying and condensing the virus suspension by sucrose density-gradient centrifugation and the like. Further, to the above virus suspension or fractionated virus fluid, formalin may be added to yield a final concentration of about 0.001 to 0.5% by volume, preferably about 0.02% by volume, and thimerosal may be added to yield a final concentration of about 0.01% by volume. Then, the resulting solution may be used as the material for the high-speed collision treatment immediately or after inactivation at about 4°C for about 1 to 14 days. Furthermore, a surfactant (for example, Polysorbate 80) may be added to the above material for the high-speed collision treatment at a final concentration of about 0.01 to 1.0% by volume, preferably about 0.1% by volume, if desired, and then the resulting mixture may be used immediately or after standing at 4°C for about 1 to 7 days.

As apparatus used for the high-speed collision treatment, any apparatus may be employed as long as it employs the principle of colliding the virus particles with an object at high speed. In collision chambers of the apparatus, for example, the virus particles may be collided with one another or with walls of the apparatus at high speed. In particular, an apparatus employing the principle of colliding the virus particles with one another is desirably employed.

The above principle will hereinafter be described in more detail.

The material for the high-speed collision treatment is supplied to the collision chamber at high pressure, preferably at high pressure induced by an air pump. The pressure can be converted to a flow rate by decreasing the diameter of a passageway. Further, it becomes possible to collide the virus particles with one another in the collision chamber by arranging outlets of two divided passageways so as to be opposite to each other. In the collision chamber, the virus particles are disrupted by an interaction of an impact produced when the virus particles collide with an object at high speed with cavitation due to vacuum action induced when the material (fluid) for the high-speed collision treatment passes at high speed. At this time, it is desirable that the material for the high-speed collision treatment maintains a constant flow rate at high pressure. For this purpose, it is preferred to use a pressure pump continuously operable at high pressure. The material for the high-speed collision treatment may be continuously circulated to disrupt the virus particles, if desired.

Examples of the apparatus employing the above principle include a Microfluidizer (registered trade mark, Microfluidics Corp. U.S.A.) and a Nanomizer (registered trade mark, Atsuryu Kogyo Co., Japan). The pressure applied to the apparatus used for the high-speed collision treatment and the flow rate of the material for the high-speed collision treatment containing the virus particles depend on the type of virus particles used for the disruption and on the desired degree of the disruption of the virus particles. The virus particles can usually be disrupted at a pressure of about 1,500 to 3,000 kg/cm² and a flow rate of about 500 to 800 m/sec. If influenza virus particles are disrupted for the purpose of manufacturing the influenza vaccine, the influenza virus particles are collided with one another, for example, at a pressure of about 1,500 to 2,500 kg/cm² and a flow rate of 500 to 700 m/sec, and continuously circulated through the apparatus for about 0.5 to 5 hours, thereby obtaining a desired disrupted product. When the influenza virus particles are collided with one another at higher speed by the application of higher pressure or by the reduction in the diameter of the passageways in the apparatus, the treating time can be shortened.

According to the disrupting method of the virus particles of the present invention, the virus particles can be disrupted leaving the partial structure of the envelopes of the virus particles unruptured. In the example directed to influenza virus particles, the influenza virus particles can be disrupted without damaging the

3

partial structure of the viral envelopes each of which consists of hemoagglutinin (HA), neuraminidase (NA), a lipid bilayer and a matrix protein. Using the resulting disrupted product, the influenza vaccine can be manufactured whose pyrogenicity, a side effect, disappears and which has the immunogenicity (potency) of HA and NA equivalent to that of the whole particle vaccine due to adjuvant action resulting from preservation of the bonding of HA and NA to the lipid bilayer and the matrix protein.

The present invention will be described in more detail with the following Examples and Experimental Example. It is understood of course that these are not intended to limit the scope of the invention.

Example 1

A/Yamagata/120/86/(H1N1) strain (an influenza virus strain for manufacturing the influenza vaccine) was inoculated in a chorioallantoic cavity of a grown egg and cultivated to obtain chorioallantoic fluid containing propagated virus. Then, the chorioallantoic fluid containing propagated virus was purified and condensed by purified sucrose density-gradient centrifugation to collect a virus fraction.

Formalin was added to the fractionated virus fluid to yield a final concentration of 0.02% by volume and the resulting mixture was allowed to stand in a cold room at 4°C for a week (7 days), thereby inactivating the virus. 75 ml of fractionated virus fluid containing inactivated intact virus (whole particle) (virus titer: 7,712 CCA/ml) particles was treated to disrupt the particles by using the Microfluidizer (registered trade mark) as follows.

The sample of the fractionated virus fluid was placed in a vessel of the Microfluidizer (registered trade mark), and then supplied into an interaction chamber by adjusting the pressure of a compressor to 7.5 to 8.0 kg/cm² and actuating a pressure pump at a pressure of 1,800 kg/cm². The virus particles were collided with one another at a flow rate of about 600 m/sec to disrupt the virus particles. After the treatment in the chamber, the sample was returned to the initial vessel again and circulated by the continuous operation of the pressure pump, thereby continuing the disruption treatment of the virus particles. The treating time to disrupt the virus particles was 120 minutes.

The resulting solution was used as original fluid of a Microfluidizer-treated vaccine. In order to determine the quality of the influenza vaccine, (1) original fluid of an existing ether-treated vaccine (HA vaccine) and (2) original fluid of an inactivated intact vaccine (whole particle vaccine) obtained by inactivating the vaccine with formalin prior to ether treatment were prepared from the above fractionated virus fluid as controls.

Example 2

A/Yamanashi/2/77(H3N2) strain (an influenza virus strain for manufacturing the influenza vaccine) was inoculated in a chorioallantoic cavity of a grown egg and cultivated to obtain chorioallantoic fluid containing propagated virus. Then, the chorioallantoic fluid containing propagated virus was purified and condensed by purified sucrose density-gradient centrifugation to collect a virus fraction.

Formalin was added to the fractionated virus fluid to yield a final concentration of 0.02% by volume and the resulting mixture was allowed to stand in a cold room at 4°C for a week (7 days), thereby inactivating the virus. 105 ml of fractionated virus fluid containing inactivated intact virus (whole particle) (virus titer: 16,372 CCA/ml) was treated to disrupt the virus particles by using the Microfluidizer (registered trade mark).

The sample of the fractionated virus fluid was placed in a vessel of the Microfluidizer (registered trade mark), and then supplied into an interaction chamber by adjusting the pressure of a compressor to 7.5 to 8.0 kg/cm² and actuating a pressure pump at a pressure of 1,704 kg/cm². The virus particles were collided with one another at a flow rate of about 600 m/sec to disrupt the virus particles. After the treatment in the chamber, the sample was returned to the initial vessel again and circulated by the continuous operation of the pressure pump, thereby continuing the disruption treatment of the virus particles. The treating time it took to disrupt the virus particles was 60 minutes.

The resulting solution was used as original fluid of a Microfluidizer-treated vaccine. In order to determine the quality of the influenza vaccine, (1) original fluid of an existing ether-treated vaccine (HA vaccine) and (2) original fluid of an inactivated intact vaccine (whole particle vaccine) obtained by inactivating the vaccine with formalin prior to ether treatment were prepared from the above fractionated virus fluid as controls.

Example 3

B/Gifu/2/73 strain (an influenza virus strain for manufacturing the influenza vaccine) was inoculated in a chorioallantoic cavity of a grown egg and cultivated to obtain chorioallantoic fluid containing propagated virus. Then, the chorioallantoic fluid containing propagated virus was purified and condensed by purified sucrose density-gradient centrifugation to collect a virus fraction.

Formalin was added to the fractionated virus fluid to yield a final concentration of 0.02% by volume and the resulting mixture was allowed to stand in a cold room at 4°C for a week (7 days), thereby inactivating the virus. 60 ml of fractionated virus fluid containing inactivated intact virus (whole particle) (virus titer: 13,080 CCA/ml) was treated to disrupt the virus particles by using the Microfluidizer (registered trade mark).

The sample of the fractionated virus fluid was placed in a vessel of the Microfluidizer (registered trade mark), and then supplied into an interaction chamber by adjusting the pressure of a compressor to 7.5 to 8.0 kg/cm$^2$ and actuating a pressure pump at a pressure of 1,800 kg/cm$^2$. The virus particles were collided with one another at a flow rate of about 600 m/sec to disrupt the virus particles. After the treatment in the chamber, the sample was returned to the initial vessel again and circulated by the continuous operation of the pressure pump, thereby continuing the disruption treatment of the virus particles. The treating time to disrupt the virus particles was 80 minutes.

The resulting solution was used as original fluid of a Microfluidizer-treated vaccine. In order to determine the quality of the influenza vaccine, (1) original fluid of an existing ether-treated vaccine (HA vaccine) and (2) original fluid of an inactivated intact vaccine (whole particle vaccine) obtained by inactivating the vaccine with formalin prior to ether treatment were prepared from the above fractionated virus fluid as controls.

Example 4

A/Fukuoka/C29/85(H3N2) strain (an influenza virus strain for manufacturing the influenza vaccine) was treated as in Example 2 to prepare original fluid of a Microfluidizer-treated vaccine, original fluid of an ether-treated vaccine (HA vaccine) and original fluid of an inactivated intact vaccine (whole particle vaccine), respectively.

Example 5

Preparation of Influenza Vaccine (Final Bulk)

The original fluids of vaccine of the virus strains (A/Yamagata/120/86(H1N1) strain, A/Yamanashi/2/77-(H3N2) strain and B/Gifu/2/73 strain) obtained in Examples 1 to 3 were mixed and diluted with phosphate buffered saline to yield CCA contents shown in Table 1, and 300 ml of a three-strain mixed influenza vaccine (final bulk) was prepared in accordance with the prescription shown in Table 1. As controls, three-strain mixed influenza vaccines (final bulks) (300 ml) were similarly prepared from original fluid of the existing ether-treated vaccine (HA vaccine) and original fluid of the inactivated intact vaccine (whole particle vaccine).

Table 1

| Composition (three strains mixed) of influenza Vaccine (final bulk) | |
|---|---|
| Composition | Final concentration |
| A/Yamagata/120/86(H1N1) strain original fluid | Amount corresponding to 200 CCA/ml |
| A/Yamanashi/2/77(H3N2) strain original fluid | Amount corresponding to 200 CCA/ml |
| B/Gifu2/73 strain original fluid | Amount corresponding to 300 CCA/ml |
| Thimerosal | 0.01 w/v% |
| Formalin | 0.004 v/v% |
| Gelatin | 0.05 w/v% |
| Polysorbate 80 | 0.01 v/v% |
| Phosphate buffered saline | M/100 |
| Hydrogen ion concentration (pH) | 7.0 - 7.2 |
| Purified saccharose | 5 w/v% or less |

Experimental Example 1

Results of quality tests of influenza vaccines

(1) Pyrogenicity of influenza vaccines

For the purpose of determining the viral pyrogenicity with respect to the original fluids of the virus strains obtained in Examples 1 to 3, pyrogenicity tests (rabbit) were carried out. The body temperatures of the rabbits were measured after elapses of 1.0, 1.5, 2.0, 2.5, 3.0, 4.0 and 5.0 hours after the inoculation of each original fluid. The results are as shown in Table 2.

As apparent from Table 2, the inactivated intact vaccines (whole particle vaccines) prior to the ether treatment showed the viral pyrogenicity on an inoculation amount of 30 to 300 CCA/kg, though it was slightly different depending on the kind of virus strain. Each strain of the existing ether-treated vaccines (HA vaccines) was negative even on an inoculation amount of 1,000 CCA/kg. In contrast, the Microfluidizer-treated vaccines did not show viral pyrogenicity and were negative even on inoculation amounts of 1,000 CCA/kg and 3,000 CCA/kg. Compared to the inactivated intact vaccines, these vaccines showed reduced pyrogenicity by 10 to 100 times or more, and the viral pyrogenicity thereof disappeared.

Table 2  Results of pyrogenicity test (rabbit) of influenza vaccines

| | | Microfluidizer-treated vaccine | | | | Ether-treated vaccine (HA vaccine) | | | |
|---|---|---|---|---|---|---|---|---|---|
| Inoculation amount(CCA/kg) | | 3,000 | | 1,000 | | 1,000 | | 3) 300 | |
| Rise of body temp. ($^{o}$C) | | 1) Max. rise | 2) Deci- sion | Max. rise | Deci- sion | Max. rise | Deci- sion | Max. rise | Deci- sion |
| Name of virus strainb | Rab- bit No. | | | | | | | | |
| A/Yama gata/ 120/86 (H1N1) | No.1 | 0.0 | – | 0.3 | –· | 0.3 | – | | |
| | No.2 | 0.2 | – | 0.4 | – | 0.3 | – | | |
| | No.3 | 0.0 | – | 0.3 | – | 0.2 | – | | |
| | (Total) | 0.2 | Suit- able | 1.0 | Suit- able | 0.8 | Suit- able | | (Suit- able) |
| A/Yama nashi/ 2/77 (H3N2 | No.1 | 0.3 | – | 0.4 | – | 0.1 | – | | |
| | No.2 | 0.2 | – | 0.0 | – | 0.3 | – | | |
| | No.3 | 0.1 | – | 0.2 | – | 0.0 | – | | |
| | (Total) | 0.6 | Suit- able | 0.6 | Suit- able | 0.4 | Suit- able | | (Suit- able) |
| B/Gifu /2/73 | No.1 | 0.4 | – | 0.3 | – | 0.2 | – | | |
| | No.2 | 0.3 | – | 0.3 | – | 0.2 | – | | |
| | No.3 | 0.2 | – | 0.3 | – | 0.4 | – | | |
| | (Total) | 0.9 | Suit- able | 0.9 | Suit- able | 0.8 | Suit- able | | (Suit- able) |

(to be continued)

Table 2   Results of pyrogenicity test (rabbit)
of influenza vaccines(continued)

| Sample | | Inactivated intact vaccine (whole particle vaccine) | | | | | |
|---|---|---|---|---|---|---|---|
| Inoculation amount(CCA/kg) | | 300 | | 100 | | 30 | |
| Rise of body temp. ($^{\circ}$C) | | Max. rise | Deci-sion | Max. rise | Deci-sion | Max. rise | Deci-sion |
| Name of virus strain | Rabbit No. | | | | | | |
| A/Yama gata/ 120/86 (H1N1) | No.1 | 0.6 | + | 0.4 | − | | |
| | No.2 | 0.7 | + | 0.3 | − | NT | |
| | No.3 | 0.6 | + | 0.1 | − | | |
| | (Total) | 1.9 | Unsuit-able | 0.8 | Suit-able | | |
| A/Yama nashi/ 2/77 (H3N2 | No.1 | | | 1.8 | + | 0.2 | − |
| | No.2 | 4) NT | | 1.6 | + | 1.4 | + |
| | No.3 | | | 1.1 | + | 0.5 | − |
| | (Total) | | | 4.5 | Unsuit-able | 2.1 | 5) |
| B/Gifu /2/73 | No.1 | NT | | 0.4 | − | 0.4 | − |
| | No.2 | | | 0.6 | + | 0.3 | − |
| | No.3 | | | 0.8 | + | 0.2 | − |
| | (Total) | | | 1.8 | 5) | 0.9 | Suit-able) |

Note: 1) This indicates the maximum temperature rise from

the body temperature before inoculation to that after

inoculation.

2) The decision mark of one rabbit is + (0.6°C or higher) or - (0.5°C or lower). (Total) is decided on the basis of minimum requirements for biological products in Japan.

3) The ether-treated vaccines (HA vaccines) are the existing influenza HA vaccines which are decided "suitable" based on an inoculation amount of 300 CCA/kg.

4) Not measured.

5) It is necessary to test again.

(2) Potency of influenza vaccines

An influenza vaccine (final bulk) having a composition similar to that in Example 5 was prepared from the original vaccine fluid of the virus strains obtained in Examples 1 to 3. As controls, three-strain mixed influenza vaccines (final bulks) were similarly prepared from the existing original fluids of ether-treated vaccine (HA vaccine) and the original fluids of the inactivated intact vaccine (whole particle vaccine). For each of the influenza vaccines (final bulks), the neutralizing antibody productivity in mice was measured with time. Namely, neutralization test in eggs (incubation time: 30 minutes) were carried out after elapses of 1, 2, 3, 4 and 5 weeks after immunization (vaccine diluent: M/100 phosphate buffer saline) of mice to determine the $ED_{50}$ values. The results are as shown in Tables 3(1) to 3(3).

As apparent from Table 3(1), the antibody production of the Microfluidizer-treated vaccine of A/Yamagata/120/86(H1N1) strain reached a peak after about 2 weeks, and the titer thereof was equivalent to, or higher than that of the inactivated intact vaccine. In contrast, the potency of the ether-treated vaccine was a little lower, and the antibody production reached a peak after 3 weeks.

As apparent from Table 3(2), the antibody production of the Microfluidizer-treated vaccine of A/Yamanashi/2/77(H3N2) strain reached a peak after 3 weeks, and the potency thereof was approximately equivalent to that of the inactivated intact vaccine. In contrast, the potency of the ether-treated vaccine was low, and the antibody production reached a peak after 4 weeks.

As apparent from Table 3(3), the antibody production of the Microfluidizer-treated vaccine of B/Gifu/2/73 strain was low in its absolute value and reached a peak after 5 weeks. However, the titer thereof was equivalent to that of the inactivated intact vaccine. In contrast, the $ED_{50}$ value of potency of the ether-treated vaccine was $5^{1.5}$ even after 5 weeks, and the antibody titer thereof was very low.

Each of the Microfluidizer-treated vaccines (final bulks) passed all the test items of minimum requirements for biological products in Japan of the existing ether-treated vaccines (HA vaccines).

Table 3(1)

| Results of potency tests (mouse) of influenza vaccine | | | | | |
|---|---|---|---|---|---|
| Name of virus | A/Yamagata/120/86(H1N1) | | | | |
| Content of vaccine | Amount corresponding to 200 CCA/ml | | | | |
| Immune period of mouse | 1W | 2W | 3W | 4W | 5W |
| Sample | | | | | |
| Microfluidizer-treated vaccine | 1.50 | 4.50 | 5.00 | 5.00 | 4.33 |
| Ether-treated vaccine (HA vaccine) | 1.67 | 3.67 | 4.50 | 3.88 | 3.80 |
| Inactivated intact vaccine (whole particle vaccine) | 1.55 | 4.50 | 4.33 | 4.13 | 4.00 |
| Dose of challenge virus | (4.67) | (4.88) | (4.67) | (4.37) | (4.67) |
| Note: | | | | | |
| 1) The potency ($ED_{50}$) is indicated by X of $5^X$. | | | | | |
| 2) Dose of challenge virus ($EID_{50}/0.2$ ml) is indicated by n of $10^n$. | | | | | |

Table 3(2)

| Results of potency tests (mouse) of influenza vaccine | | | | | |
|---|---|---|---|---|---|
| Name of virus | A/Yamanashi/2/77(H3N2) | | | | |
| Content of vaccine | Amount corresponding to 200 CCA/ml | | | | |
| Immune period of mouse | 1W | 2W | 3W | 4W | 5W |
| Sample | | | | | |
| Microfluidizer-treated vaccine | ≦0.50 | 3.00 | 4.50 | 4.64 | 4.33 |
| Ether-treated vaccine (HA vaccine) | ≦0.50 | 2.00 | 4.33 | 4.50 | 3.88 |
| Inactivated intact vaccine (whole particle vaccine) | 1.00 | 3.67 | 5.00 | 4.13 | 4.00 |
| Dose of challenge virus | (4.20) | (4.20) | (3.67) | (4.50) | (4.29) |
| Note: | | | | | |
| 1) The potency ($ED_{50}$) is indicated by X of $5^X$. | | | | | |
| 2) Dose of challenge virus ($EID_{50}/0.2$ ml) is indicated by n of $10^n$. | | | | | |

EP 0 374 817 A2

Table 3(3)

| Results of potency tests (mouse) of influenza vaccine | | | | | |
|---|---|---|---|---|---|
| Name of virus | B/Gifu/2/73 | | | | |
| Content of vaccine | Amount corresponding to 200 CCA/ml | | | | |
| Immune period of mouse | 1W | 2W | 3W | 4W | 5W |
| Sample | | | | | |
| Microfluidizer-treated vaccine | $\leq 0.50$ | $\leq 0.50$ | 2.50 | 3.00 | 3.67 |
| Ether-treated vaccine (HA vaccine) | $\leq 0.50$ | $\leq 0.50$ | 0.71 | 1.50 | 1.50 |
| Inactivated intact vaccine (whole particle vaccine) | $\leq 0.50$ | $\leq 0.50$ | 3.20 | 3.5 | 4.00 |
| Dose of challenge virus | (4.67) | (3.50) | (3.67) | (3.67) | (4.33) |
| Note: | | | | | |
| 1) The potency ($ED_{50}$) is indicated by X of $5^X$. | | | | | |
| 2) Dose of challenge virus ($EID_{50}$/0.2 ml) is indicated by n of $10^n$. | | | | | |

## Claims

1. A method for disrupting virus particles which comprises subjecting the virus particles to high-speed collision treatment.

2. A method according to claim 1 wherein the virus particles are collided with one another.

3. A method according to claim 2 wherein the high-speed collision treatment is carried out at a flow rate of about 500 to 800 m/sec.

4. A method according to claim 2 wherein the high-speed collision treatment is carried out at a pressure of about 1,500 to 3,000 kg/cm² and a flow rate of about 500 to 800 m/sec.

5. A method according to claim 4 wherein the virus particles are virus particles having a diameter of about 18 to 600 nm.

6. A method according to claim 1 wherein the virus particles are virus particles selected from the class consisting of influenza virus particles, rubella virus particles, Japanese encephalitis virus particles, mumps virus particles, measles virus particles, adenovirus particles, herpes simplex virus particles, rabies virus particles, vaccinia virus particles, hepatitis B virus particles, hepatitis A virus particles, adult T cell leukemia virus particles and human immunodeficiency virus particles.

7. A method according to claim 1 wherein the virus particles are influenza virus particles.

8. A method according to claim 7 wherein the influenza virus particles are collided with one another.

9. A method according to claim 8 wherein the high-speed collision treatment is carried out at a flow rate of about 500 to 700 m/sec.

10. A method according to claim 7 wherein the high-speed collision treatment is carried out at a pressure of about 1,500 to 2,500 kg/cm² and a flow rate of about 500 to 700 m/sec.

11

Fig. 1

Fig. 2

Fig. 3